# EUROPÄISCHE PATENTSCHRIFT

(11) **EP 1 245 238 B1**
(45) Veröffentlichungstag und Bekanntmachung des Hinweises auf die Patenterteilung: **19.09.2007**
(21) Anmeldenummer: 02004217.2
(22) Anmeldetag: 25.02.2002
(51) Int. Cl.: A61K 47/48, C07K 14/31

(54) **Exotoxin-Ligand**
Exotoxin ligand
Ligand exotoxine

(30) Priorität: 30.03.2001 DE 10116042
(43) Veröffentlichungstag der Anmeldung: 02.10.2002
(73) Patentinhaber: Fresenius Medical Care Deutschland GmbH, 61352 Bad Homburg (DE)
(72) Erfinder: Maschke, Hans E., Dr., 61352 Bad Homburg (DE); Otto, Veit, Dr., 66606 St. Wendel (DE)
(74) Vertreter: Müller-Boré & Partner Patentanwälte

(56) Entgegenhaltungen:
- EP-A- 0 424 698
- EP-A- 0 993 834
- EP-A- 1 191 034
- WO-A-89/01335
- WO-A-94/25483
- WO-A-98/29444
- DE-A- 19 705 366
- JETT M ET AL: "IDENTIFICATION OF STAPHYLOCOCCAL ENTEROTOXIN B SEQUENCES IMPORTANT FOR INDUCTION OF LYMPHOCYTE PROLIFERATION BY USING SYNTHETIC PEPTIDE FRAGMENTS OF THE TOXIN" INFECTION AND IMMUNITY, AMERICAN SOCIETY FOR MICROBIOLOGY. WASHINGTON, US, Bd. 62, Nr. 8, 1. August 1994 (1994-08-01), Seiten 3408-3415, XP002062764 ISSN: 0019-9567

## Beschreibung

Die vorliegende Erfindung betrifft einen Verfahren zur Herstellung eines Adsorbens mit eine Liganden für Bakterientoxine, insbesondere Entero- bzw. Exotoxine grampositiver Bakterien, der zur selektiven Wechselwirkung mit einer Struktur befähigt ist, die eine Aminosäuresequenz umfaßt, welche in den Bakterientoxinen konserviert ist.

Bakterielle Superantigene gehören zu den stärksten Toxinen. Zu der Familie der Superantigene gehören beispielsweise die Entero- bzw. Exotoxine grampositiver Bakterien wie SEA bis SEE (wobei SEB am häufigsten auftritt) aus *Staphylococcus,* das toxische Schocksyndrom Toxin 1 (TSST-1), sowie SPEA und SPEC aus *Streptococcus.* Diese regen durch Bindung an den T-Zellrezeptor (TCR) und/oder den Major Histocompatibility Complex II (MHC II) der T-Helferzellen 1 (Th1) die Produktion und Ausschüttung von Lymphokinen (bzw. Cytokinen oder Interleukinen) wie beispielsweise IL-2, IFN-γ und/oder TNF-β an. Als Superantigene wirken diese Moleküle entkoppelt vom normalen Mechanismus der Aktivierung der Immunantwort über die Präsentation von prozessierten Antigenen.

Zur Behandlung bzw. Verhinderung eines durch derartige Superantigene ausgelösten toxischen Schocks sind im Stand der Technik u.a. Materialien vorgeschlagen worden, um diese pyrogen wirkenden Substanzen aus dem Blut eines Patienten zu entfernen. So sind in US-A-4,381,239 Adsorbenzien für Pyrogene beschrieben worden, die einen wasserunlöslichen Träger und eine stickstoffhaltige, heterozyklische Verbindung umfassen. In US-A-5,928,633 wird ein Material, enthaltend eine Harnstoff- oder Thioharnstoff-Bindung, offenbart, welches eine Affinität zum *Staphylococcus*-Enterotoxin und *Streptococcus-*Exotoxin aufweist. Weiterhin ist in DE-A-197 05 366 eine Vorrichtung zur Reinigung proteinhaltiger Lösungen, wie Blut, Blutplasma oder Kulturmedien, beschrieben, die ein bioverträgliches Trägermaterial aus Kunststoff umfaßt, das mittels Peptidbindungen mit Albumin kovalent beschichtet ist und dadurch eine Reihe von Toxinen, beispielsweise exogene Toxine, binden soll.

Die im Stand der Technik bekannten Adsorbenzien enthaltenen Liganden binden jedoch relativ unspezifisch an unterschiedlichste Stellen der zu bindenden Proteinstrukturen und/oder weisen nur eine Affinität zu einzelnen Vertretern der Superantigene auf.

Der vorliegenden Erfindung liegt somit die Aufgabe zugrunde, ein neues System zur Bindung von Bakterientoxinen bereitzustellen, das auf der selektiven Wechselwirkung mit den in den Toxinen konservierten Strukturen beruht und daher in pharmazeutischen Zusammensetzungen und Adsorbenzien verwendbar ist, um beispielsweise einen von derartigen Bakterientoxinen hervorgerufenen bzw. zu befürchtenden septischen Schock zu behandeln bzw. zu verhindern.

Diese Aufgabe wird durch die in den Ansprüchen gekennzeichneten Ausführungsformen der vorliegenden Erfindung gelöst. Insbesondere wird diese Aufgabe durch das in Anspruch 1 definierte Verfahren zur Herstellung eines Absorbens gelöst.

Der Ligand für Bakterientoxine ist zur selektiven Wechselwirkung mit der β-Faltblatt-Hinge-α-Helix-Struktur der Superantigene befähigt.

Der Ausdruck "zur selektiven Wechselwirkung befähigt" bedeutet, daß der erfindungsgemäße Ligand eine hohe Affinität zu der in den Toxinen konservierten Struktur aufweist und daher bevorzugt mit dieser wechselwirkt. Vorzugsweise beträgt die Assoziationskonstante des Liganden für das Toxin bzw. die Struktur, z.B. *in vitro* unter physiologischen oder näherungsweise physiologischen Bedindungen, wie bei 37°C, pH 7,4 und etwa 140 bis 150 mM NaCI, mindestens 10⁶ M⁻¹, mehr bevorzugt mindestens 10⁸ M⁻¹. Die Wechselwirkung des Liganden kann dabei jegliche chemischen bzw. physikalischen Bindungsarten, wie beispielsweise elektrostatische Wechselwirkungen, Van-der-Waals-Wechselwirkungen, Wasserstoffbrückenbindungen und/oder hydrophobe Wechselwirkungen, umfassen.

Die Bereitstellung des Liganden beruht somit auf der Erkenntnis, daß die zu den Superantigenen gehörenden Bakterientoxine zwar untereinander nur eine geringe Homologie bezüglich ihrer Gesamtsequenzen aufweisen, jedoch innerhalb dieser Sequenzen konservierte Sequenzmotive vorliegen bzw. konservierte Raumstrukturen durch diese zusammenhängenden Aminosäuresequenzen und/oder in der linearen Sequenz entfernt voneinander vorliegenden Aminosäurereste ausgebildet werden.

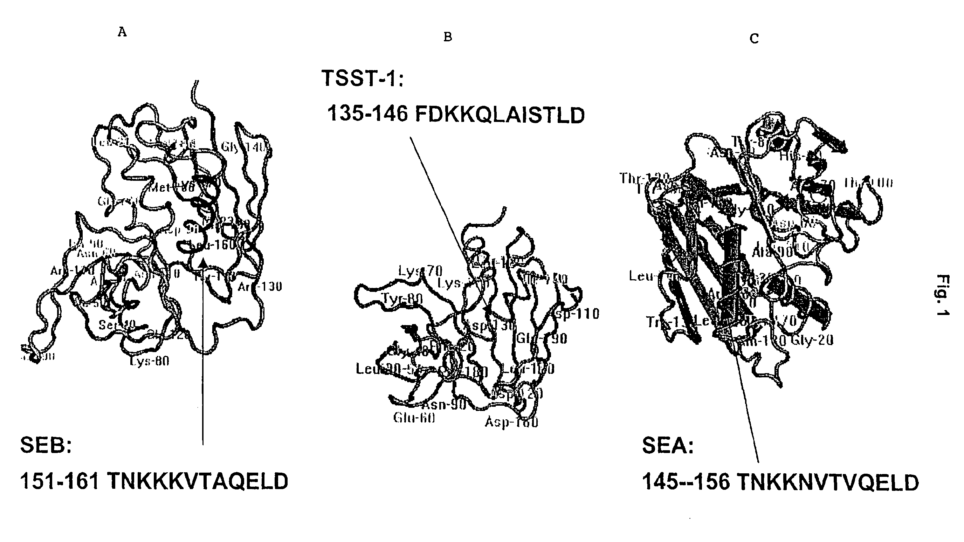

Insbesondere ist die β-Faltblatt-Hinge-α-Helix-Struktur der Superantigene, auch als P12 bezeichnet, stark konserviert (vgl. Fig. 1) und umfaßt im allgemeinen ein Dodekapeptid. Die entsprechende Sequenz von SEB umfaßt die Aminosäuren 150 bis 161 und lautet TNKKKVTAQELD (SEQ ID NO 1). Das entsprechende Strukturmotiv in SEA (TNKKNVTVQELD, Aminosäuren 145 bis 156, SEQ ID NO 2) ist im Vergleich zu SEB nur in zwei Positionen verändert. Der entsprechende Sequenzbereich von TSST-1 (FDKKQLAISTLD, Aminosäuren 135 bis 146, SEQ ID NO 3) weist zwar im Vergleich zu SEB lediglich vier identische Reste auf, bildet jedoch, wie die in Fig. 1 rot markierte Formation zeigt, eine sehr ähnliche Raumstruktur aus. Die vorstehenden Aminosäurenummerierungen beziehen sich auf die von Papageorgiou et al. (Protein Sci. 5 (8), 1737-1741, 1996), Sundstrom et al. (J. Biol. Chem. 271, 32212-32216, 1996) bzw. Papageorgiou et al. (J. Mol. Biol. 277, 61-79, 1998) publizierten Sequenzen.

Auf eine mögliche Bedeutung dieser konservierten Struktur für die Untersuchung und Wirkungsweise der betreffenden Bakterientoxine wurde in Arad et al.. (2000), Nature Medicine 6, 414 - 421, hingewiesen. Danach inhibiert das Dodekapeptid YNKKKVTAQELD (SEQ ID NO 4), bei dem im Vergleich zum Ausgangssequenzmotiv von SEB das erste Threonin gegen Tyrosin ausgetauscht wurde, da die entsprechende erste Aminosäure bei TSST-1 ein Phenylalanin ist, als Antagonist die Aktivierung der Immunantwort, d.h. die Lymphokinproduktion. Durch die intravenöse Verabreichung des Peptids gelang es im Tierversuch bei Mäusen, einen septischen Schock zu verhindern. Dabei wurde auch die Inhibition der Lymphokinproduktion hinsichtlich der durch SEA, SEB, TSST-1 und SPEA modulierten Immuninduktion gezeigt. Allerdings offenbaren Arad et al. nicht die Möglichkeit, gegen eine in den Bakterientoxinen konservierte Struktur, beispielsweise die β-Faltblatt-Hinge-α-Helix-Domäne, gerichteten Liganden bereitzustellen.

Vorzugsweise bindet somit der Ligand Exo- bzw. Enterotoxine grampositiver Bakterien, wie SEA, SEB, SEC, SED, SEE, TSST-1, SPEA und SPEC.

Gemäß bevorzugten Ausführungsformen kann der Ligand ein synthetischer organischer Ligand, ein Saccharid, beispielsweise ein Oligosaccharid, ein Peptid, beispielsweise ein Oligopeptid oder eine Nukleinsäure, wie ein einzelsträngiges Oligonukleotid, sein. Ein Beispiel eines Peptid-Liganden ist ein Antikörper, der gegen die konservierte Struktur der Superantigene gerichtet ist. Dabei kann der Antikörper monoklonal, polyklonal, rekombinant sein oder er kann eine Chimäre, beispielsweise aus einem variablen Anteil der Maus und invarianten Anteilen aus Mensch, sein.

Hinsichtlich möglicher immunologischer Probleme, insbesondere wenn der Ligand in einem Adsorbens enthalten ist, ist es bevorzugt, daß der Ligand ein Oligopeptid, Oligosaccharid oder Oligonukleotid umfaßt, wobei es sich mehr bevorzugt um solche Oligomere mit nicht mehr als 100 Bausteinen handelt.

Nachfolgend werden Verfahren zur Herstellung des Liganden beschrieben.

Zur Herstellung des Liganden können unterschiedliche Verfahren verwendet werden, wobei bei der Auswahl des konkreten Verfahrens die folgenden Punkte zu beachten sind:
- thermische Stabilität der Liganden sowie Stabilität gegenüber der biologischen Umgebung (z.B. Blut oder Blutplasma),
- große Variabilität der Liganden zur Abdeckung einer großen Zielmolekülgruppe während der Ligandenidentifizierung,
- Wirtschaftlichkeit des Verfahrens und
- ausreichende Affinität der Liganden zur Zielstruktur, um in der Absorption aus Blut oder Blutplasma verwendet zu werden.

So können beispielsweise synthetische Liganden unter Verwendung der sogenannten SPOT-Synthese identifiziert werden. Die SPOT-Synthese (oder Fleck-Synthese) bietet die Möglichkeit, durch automatisierte Parallelsynthese Peptidbibliotheken auf Festphasen schnell und flexibel herzustellen. Das Prinzip besteht in der Verteilung kleinster Tropfen mit Lösungen von Reagenzien auf vorher genau definierte Bereiche (engl. "spots", Flecke) einer geeigneten Oberfläche (beispielsweise Cellulosemembranen, Glas usw.). Die Fixierung an einer gemeinsamen Oberfläche bietet den Vorteil, daß die entstehenden Bibliotheken in einem einfach zu handhabenden Format vorliegen. Die Zwischenschritte der Synthese, wie Waschen, oder die Entfernung von Schutzgruppen, können für alle Mitglieder der Bibliothek gemeinsam durchgeführt werden. Darüber hinaus kann die Bibliothek in dieser Form auch gemeinsam gescreent werden, was insgesamt eine deutliche Zeit- und Materialersparnis mit sich bringt.

Die SPOT-Technik kann durch die Anwendung eines baukastenartigen Systems, das von Jerini et al., *supra,* entwickelt wurde, zum Aufbau verschiedenster Ligandensysteme verwendet werden. Die zu bindende Leitstruktur, beispielsweise eine Struktur, welche die Aminosäuresequenz TNKKKVYAQELD (SEQ ID NO 1) umfaßt, kann je nach den Anforderungen optimiert bzw. stabilisiert werden. Neben der Durchführung von Mutationen mit den natürlichen Aminosäuren kann an jeder Stelle der Sequenz ein beliebiger Baustein eingeführt werden. Es steht ein umfangreiches Repertoire an organisch-synthetischen Bausteinen zu Verfügung, die einem Fachmann bekannt sind. Beispielsweise können 1,3,5-Trichlortriazinbausteine verwendet werden, deren Einführung durch die Möglichkeit kontrollierter Mehrfachsubstitution zahlreiche Modifikationsmöglichkeiten bereitstellt. Durch schrittweise Substitution aller Aminosäuren kann so auch ein vollsynthetischer, organischer Ligand aufgebaut werden. Dieser Sachverhalt wird durch das folgende Reaktionsschema veranschaulicht:

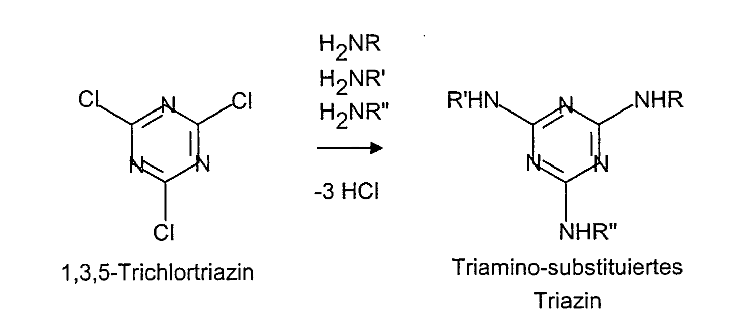

Derartige synthetische Liganden sind in biologischer Umgebung wie Blut und Blutplasma stabil.

Selbstverständlich kann das gewünschte Peptid *per se* durch im Stand der Technik bekannte Verfahren chemisch modifiziert bzw. derivatisiert werden.

Ein weiteres Verfahren zur Bereitstellung von Liganden auf Peptidbasis bedient sich der sogenannten Phagen-Display-Technik. Dabei wird durch Manipulation der DNA eines Phagen eine zusätzliche Peptidsequenz auf der Oberfläche dieses Phagen präsentiert und somit für Bindungstests mit dem Zielmolekül zugänglich. Wird in der verwendeten Phagen-Bibliothek eine das Zielmolekül bindende Sequenz ermittelt, so kann deren Primärstruktur in bekannter Weise durch Amplifikation der entsprechenden Phagen-DNA und deren Sequenzierung aufgeklärt werden. Ein Vorteil der Phagen-Display-Technik besteht beispielsweise darin, daß ein zunächst identifizierter Ligand bei dieser Technik in einfacher Weise hinsichtlich seiner Affinität für das Zielmolekül optimiert werden kann. Dazu wird die für den Peptid-Liganden kodierende Nukleotidsequenz durch Austausch, Addition, Deletion und/oder Insertion ein oder mehrerer Nukleotide derart modifiziert (Mutagenese), daß schnell und einfach, ausgehend vom zunächst identifizierten Peptid-Liganden, ein hinsichtlich der Affinität zur Zielstruktur verbesserter Ligand bereitgestellt wird, der eine entsprechend der Manipulation der kodierenden DNA veränderte Aminosäuresequenz aufweist.

Ein Problem der ursprünglichen Phagen-Display-Technik besteht darin, daß, ähnlich wie bei kombinatorischen Peptid-Bibliotheken, Bibliotheken ab einer gewissen Anzahl von Aminosäuren nur schwierig aufzubauen sind (Eine Bibliothek aus Peptiddodekameren auf Basis aller 20 natürlich vorkommenden Aminosäuren erfordert beispielsweise mehr als 10¹⁵ Klone.). Die Verwendung kürzerer Peptide erreicht jedoch in der Regel nicht die Bindungsaffinitäten längerer Peptide. Daher wird erfindungsgemäß vorzugsweise die nachfolgend beschriebene "Cosmix-Strategie" (Cosmiplexing-Technik, Fa. Cosmix Molecular Biologicals GmbH) verwendet: In einem ersten Selektionszyklus erfolgt eine Vorauswahl von Peptidsequenzen, welche mit der Zielstruktur wechselwirken. Innerhalb der gefundenen Sequenzen wird daraufhin durch die Cosmiplexing-Technik eine Optimierung der Affinität zum Zielmolekül vorgenommen. Die Bindungsoptimierung erfolgt besonders effektiv, da durch die verwendete spezifische Kombinationstechnik eine extrem hohe Diversität in der vorausgewählten Sequenzbibliothek erzielt wird, die zu einer optimalen Kombination bindender Strukturinkrimente führt. Dies führt zu Affinitäten, insbesondere innerhalb kurzer Peptidsequenzen, die sonst nur mit höhermolekularen Oligomeren aus wesentlich größeren Grundbibliotheken erreicht wird.

Gemäß einer anderen Ausführungsform kann die Vielfalt von Phagen-Display-Bibliotheken durch die Eingrenzung der mutierbaren Ausgangssequenz begrenzt werden. Als Grundlage zum Aufbau einer Phagen-Display-Bibliothek werden beispielsweise von der Firma Dyax (Cambridge, MA, USA) Proteaseinhibitoren bereitgestellt. Teile dieser Proteine werden in der Bibliothek auf dem Phagen präsentiert, und es werden innerhalb der bindenden Domänen Mutationen einzelner oder mehrerer Aminosäuren vorgenommen. Das bei der Verwendung natürlich vorkommender Peptide bestehende Problem der möglichen Instabilität in biologischen Flüssigkeiten wie Blut oder Blutplasma kann durch die Verwendung von Liganden auf Proteaseinhibitorbasis umgangen werden, die im allgemeinen eine hohe Biostabilität aufweisen.

Zum Screening wird das Zielmolekül auf Mikrokugeln bzw. Beads immobilisiert und mit der Phagen-Bibliothek in bekannter Weise in Kontakt gebracht. Die Mikrokugeln mit den darin über die Peptid-Liganden gebundenen Phagen werden isoliert und die Phagen von den von ihnen präsentierten Peptiden enzymatisch abgespalten. Die so abgetrennten Phagen, welche die DNA-Moleküle enthalten, die für die das Zielmolekül bindenden Peptid-Liganden kodieren, werden in *E. coli* amplifiziert und stehen somit für weitere Schritte, beispielsweise die vorstehend benannte Mutagenese zur Optimierung der Affinität des Liganden zur Zielstruktur usw., zur Verfügung.

Des weiteren können aufgrund der in der Natur häufig vorkommenden spezifischen Protein-Nukleinsäure-Wechselwirkungen (z.B. Protein/RNA in Ribosomen und Protein/DNA in Nukleasen) auch Nukleinsäuren zur Herstellung des erfindungsgemäßen Liganden herangezogen werden. Bei der Verwendung von Nukleinsäuren ist über eine Basensubstitution die Bereitstellung einer großen Anzahl von Sequenz- und damit Strukturvarianten in einfacher Weise möglich. Die Sequenzen mit vordefinierter Länge werden an eine Matrix gekoppelt und, wie vorstehend hinsichtlich des Phage-Displays beschrieben, mit dem Zielmolekül in Kontakt gebracht. Dabei bindende Nukleinsäuren weisen eine Struktur auf, welche zur Wechselwirkung mit dem Zielmolekül befähigt ist. Hinsichtlich der Optimierung der Sequenz zur Bereitstellung eines Liganden mit höherer Affinität weisen Nukleinsäuren den Vorteil auf, daß sie leicht amplifizierbar sind (z.B. *in vitro* durch PCR oder *in vivo* mittels *E. coli),* weshalb als Ausgangspunkt zur Bindungsoptimierung die Bindung eines einzigen Nukleinsäuremoleküls ausreichend sein kann. Unter geeigneten Bedingungen kann eine, wie vorstehend beschriebene weitere Passage eines angereicherten (amplifizierten) ersten Nukleinsäuregemischs durchgeführt werden.

Wie bei den vorstehend beschriebenen Peptidliganden kann auch bei Nukleinsäuren das Problem der Instabilität in biologischer Umgebung auftreten. Insbesondere ist RNA häufig instabiler als DNA. Als Nukleinsäureliganden kommen einzelsträngige als auch doppelsträngige Spezies (wobei bei einem einzelsträngigen Molekül selbstverständlich intramolekulare Basenpaarungen auftreten können) in Betracht. Zur Stabilisierung gegenüber den biologischen Flüssigkeiten wie Blut oder Blutplasma vorkommenden Nukleasen sind beispielsweise die nachstehend beschriebenden Vorgehensweisen geeignet.

Ein bevorzugtes Verfahren zur Bereitstellung eines Liganden auf Nukleinsäurebasis beruht auf dem Prinzip der sog. Spiegelmertechnologie (vgl. WO-A-98/00885). Dabei wird vom Zielmolekül (beispielsweise das Dodekapeptid mit der SEQ ID NO 4) ein "Spiegelbild", d.h. dessen Enantiomer, bestehend aus L-Aminosäurebausteinen, synthetisiert. Das derart gespiegelte Zielmolekül wird nun mit einer Bibliothek, beispielsweise aus RNA-Oligomeren in deren natürlicher D-Form, gescreent. Die Sequenz bindender Bibliotheksmitglieder wird bestimmt und dann in Form ihrer nicht natürlicherweise vorkommenden L-lsomere synthetisiert. Diese L-lsomere binden aufgrund der Stereochemie an die ungespiegelten Zielmoleküle. Damit werden NukleinsäureLiganden bereitgestellt, die insbesondere für den Einsatz in biologischen Flüssigkeiten geeignet sind, da sie aufgrund der in der Natur nicht vorkommenden L-Form im wesentlichen biologisch inert sind.

Liganden auf Nukleinsäurebasis können weiterhin beispielsweise durch den Einbau Phosphat-modifizierter Nukleotide stabilisiert werden. Hierbei wird durch den Einbau entsprechend modifizierter Nukleotide, beispielsweise mittels PCR, eine Stabilisierung in biologischer Umgebung erreicht. Beispielsweise sind Substitutionen von Sauerstoff an der Phosphatgruppe durch Schwefel (vgl. ³⁵S-DNA-Sequenzierung, α-Thiophosphate) oder durch eine Methylgruppe möglich. Derart modifizierte ssDNA-Moleküle sind z.B. vor dem Abbau durch DNAsen geschützt.

Eine weitere Möglichkeit der Stabilisierung insbesondere von DNA-Liganden besteht in der Methylierung über Methylasen. So können z.B. DNA-Liganden in Bakterien wie *E. coli* unter Anwesenheit verschiedener Methylasen, die vorzugsweise Plasmid-kodiert vorliegen, hergestellt werden. Dabei werden insbesondere bei ssDNA-Liganden durch intramolekulare Basenpaarung entstandene doppelsträngige Abschnitte durch die Methylierung gegen Endonukleasen geschützt. Ein wirksamer Schutz gegenüber Exonukleasen kann darüber hinaus durch das Anfügen repetitiver "Cap"-Sequenzen erreicht werden.

Anwendungsmöglichkeiten für den vorstehend definierten Liganden umfassen beispielsweise dessen Verwendung zur Reinigung von Bakterientoxinen bzw. deren Entfernung aus Flüssigkeiten, wie beispielsweise Blut oder Blutplasma, und zur Inhibition der Zielbakterientoxine aufgrund der selektiven Bindung an eine darin vorliegende konservierte Struktur.

Die vorliegende Erfindung betrifft ein Verfahren zur Herstellung eines Adsorbens, umfassend eine Matrix, vorzugsweise eine organische Matrix, und mindestens einen kovalent an die Matrix gebundenen, vorstehend definierten Liganden.

Das Adsorbens ist vorzugsweise biokompatibel. Ein "biokompatibles" Adsorbens ist vorzugsweise blut- bzw. plasmaverträglich. Gemäß einer weiteren bevorzugten Ausführungsform ist es vollblutbverträglich.

Prinzipiell sind mehrere Trägermaterialien als Matrix denkbar, wie beispielweise Glas, Kohlenhydrate, Sepharose ®, Silica oder organische Matrices, wie Copolymere von Acrylaten oder Methacrylaten sowie Polyamiden. Vorzugsweise besteht die Matrix aus organischem Material und besonders bevorzugt sind von (Meth)acrylsäureestern und/oder -amiden abgeleitete Copolymere. Diese weisen vorzugsweise Epoxidgruppen auf. Unter dem Begriff "(Meth)acryl" sind sowohl die entsprechenden Acryl- als auch Methacrylverbindungen zu verstehen.

Als Matrix für das Absorbens am meisten bevorzugt ist ein durch Polymerisation der monomeren Einheiten
(A) (Meth)acrylamid in einer Menge von 10 bis 30 Gew.-%,
(B) N,N'-Methylen-bis(meth)acrylamid in einer Menge von 30 bis 80 Gew.-% und
(C) Allylglycidylether und/oder Glycidyl-(meth)acrylat in einer Menge von 10 bis 20 Gew.-%, jeweils bezogen auf das Gesamtgewicht der monomeren Einheiten, hergestelltes, statistisches Copolymer.

Das Copolymer wird vorzugsweise durch Suspensionspolymerisation hergestellt.

Ein derartiges Copolymer ist im Handel unter der Bezeichnung Eupergit C250L bzw. Eupergit FE162 von der Firma Röhm GmbH erhältlich.

Bei Verwendung des vorgenannten Copolymers oder einer anderen organischen Matrix, das bzw. die Oxirangruppen (Epoxidgruppen) enthält, z.B. ein im Rahmen der vorliegenden Erfindung ebenfalls bevorzugtes Copolymer, welches durch Suspensionspolymerisation von Ethylenglycoldimethacrylat und Glycidylmethacrylat und/oder Allylglycidylether erhalten wurde, werden diese Oxirangruppen vor der Einführung der kovalent anzubindenden Liganden vorzugsweise mit Ammoniak oder einem primären Amin aminiert. Dabei ist Ammoniak aus verfahrenstechnischen Gründen und aus Kostengründen bevorzugt.

Beim Einsatz des Adsorbens zur Entfernung von Bakterientoxinen, wie Entero- bzw. Exotoxinen grampositiver Bakterien, aus Blut oder Blutplasma, kann die Matrix z.B. in der Form von sphärischen, unaggregierten Partikeln, sog. Mikrokugeln bzw. Beads, Fasern oder einer Membran vorliegen, wobei eine poröse Matrix bereitgestellt wird, die eine größere Oberfläche aufweist. Die Bildung bzw. Einstellung von Porosität kann beispielsweise durch Zugabe von Porenbildnern, wie Cyclohexanol oder 1-Dodecanol zu der Reaktionsmischung der Suspensionspolymerisation für die Matrix erreicht werden. Es ist des weiteren vorteilhaft, wenn die Matrix eine Ausschlußgrenze von mindestens 10⁷ Dalton besitzt, so daß die Bakterientoxine mit dem Plasma in die Poren eindringen können, um zu den matrixgebundenen Liganden zu gelangen.

Eine weitere vorteilhafte Ausgestaltung der Erfindung liegt darin, das Adsorbens durch eine geeignete Wahl der Trägermatrix im Vollblut einzusetzen. Dazu besteht die Matrix aus unaggregierten, sphärischen Partikeln in einem Teilchengrößenbereich von 50 bis 250 µm und besitzt eine Ausschlußgrenze von mindestens 10⁷ Dalton. Dadurch können Blutzellen in Kontakt mit dem Adsorbermaterial treten, ohne daß sich die Säule zusetzt oder unzumutbar viele Zellen zurückgehalten werden oder aggregieren. Dies wird durch die Größe und die sphärische Gestalt der Beads in Verbindung mit der Ausschlußgrenze des erfindungsgemäßen Adsorbens ermöglicht, da die Zellen an der glatten äußeren Oberfläche der Beads entlanggleiten, wodurch nur eine geringe Thrombozytenadhäsion erfolgt und das Plasma mit den Bakterientoxinen dennoch die Möglichkeit hat, in die Poren einzudringen.

Dadurch entfallen extrakorporale Schritte, wie die Abtrennung von Blutzellen, das Behandeln des isolierten Plasmas und das anschließende Zusammenführen der Blutbestandteile, wodurch die Biokompatibilität des Verfahrens gesteigert wird, beispielsweise die Gefahr einer Komplementaktivierung weiter erheblich verringert wird. Das Entfallen extrakorporaler Schritte bewirkt ferner eine Verkürzung der Behandlungszeit und eine Vereinfachung des Verfahrens, wodurch eine möglichst schnelle Entfernung der Toxine aus dem Blutkreislauf des Patienten ermöglicht wird.

Das Adsorbens kann des weiteren zur Reinigung von Bakterientoxinen, vorzugsweise Entero- bzw. Exotoxine grampositiver Bakterien, verwendet werden.

Ein Verfahren zur Reinigung von Bakterientoxinen aus einem Fluid umfaßt die Schritte
a) Bereitstellen des wie vorstehend definierten sind Adsorbens und
b) Inkontaktbringen des Fluids mit dem Adsorbens.

Vorzugsweise wird das Reinigungsverfahren kontinuierlich durchgeführt, wobei das Adsorbens beispielsweise in einer Chromatographiesäule bereitgestellt und das Fluid mit den zu reinigenden Toxinen über die das Adsorbens enthaltende Chromatographiesäule in an sich bekannter Weise gegeben wird. Es ist jedoch ebenfalls möglich, das erfindungsgemäße Adsorbens in einem Batch-Verfahren zu verwenden.

Wie vorstehend ausgeführt, kann ein den Liganden für Bakterientoxine enthaltendes Adsorbens zur Erniedrigung der Konzentration von Bakterientoxinen, insbesondere der Entero- und Exotoxine grampositiver Bakterien, in Blut oder Blutplasma dienen. Hierzu wird das Adsorbens bei der Herstellung eines entsprechenden Adsorbers verwendet.

Ein Adsorber zur Erniedrigung der Konzentration von Bakterientoxinen im Blut oder Blutplasma, weist ein Gehäuse auf, welches vorzugsweise in Röhren- oder Säulenform ausgebildet ist, und welches das Adsorbens als Füllmaterial enthält. Hinsichtlich der üblicherweise durchzusetzenden Blut- bzw. Blutplasmamengen und hinsichtlich der Effizienz des Adsorbers umfaßt dieser vorzugsweise ein Volumen von 30 bis 1250 ml, mehr bevorzugt 50 bis 200 ml, insbesondere wenn es sich um einen regenerierbaren Adsorber handelt. Der Adsorber kann einzeln, im Doppel- oder Mehrfachbetrieb eingesetzt werden. Bei zwei oder mehreren Adsorbern besteht die Möglichkeit, abwechselnd einen Adsorber mit dem Blut bzw. Blutplasma zu beschicken, während der andere Adsorber regeneriert wird. Dies führt zu einer weiteren Steigerung der Effizienz beim Einsatz des Adsorbers, insbesondere da es bei der Behandlung und/oder Prävention einer grampositiven Sepsis mit dem Adsorber entscheidend sein kann, die betreffenden Toxine möglichst schnell aus dem Blut bzw. Blutplasma des Patienten zu entfernen. Der Adsorber ist vorzugsweise derart ausgebildet, daß er ein Gehäuse mit einem kopfseitigen Einlaßbereich aufweist, durch welchen das Blut oder Blutplasma dem Adsorber zugeführt wird, wobei sich in diesem Fall der Auslaß am Boden des Gehäuses des Adsorbers befindet.

Um zu verhindern, daß unerwünschte Substanzen, beispielsweise Substanzen, die vom Adsorbensmaterial herrühren, mit dem behandelten Blut bzw. Blutplasma in den Blutkreislauf des Patienten zurückgeführt werden, befindet sich vorzugsweise am Auslaß des Adsorbergehäuses angeordnet ein Filter. Dabei handelt es sich vorzugsweise um einen Partikelfilter.

Unter Verwendung des Adsorbers wird auch ein Verfahren zur Behandlung und/oder Prävention der grampositiven Sepsis bereitgestellt, bei welchem das Blut bzw. Blutplasma eines Patienten, der sich mit den betreffenden Bakterien, wie *Staphylococcus* oder *Streptococcus,* infiziert hat und bei dem möglicherweise aufgrund der von den Bakterien herrührenden Toxine ein septischer Schock zu befürchten ist, in einem Kreislauf über den erfindungsgemäßen Adsorber geführt wird.

Wie vorstehend erläutert, kann der Ligand durch seine selektive Bindung an die betreffenden Bakterientoxine auch zur Inhibition oder Herabsetzung der toxischen Wirkung dieser Moleküle verwendet werden.

Eine weitere Anwendungsmöglichkeit des Liganden besteht in seiner Verwendung zum Nachweis der betreffenden Bakterientoxine, was z.B. dazu dienen kann, eine aufgrund der Infektion mit Bakterien, wie grampositiven Bakterien, möglicherweise auftretende Sepsis aufzuspüren, um frühzeitg geeignete therapeutische Gegenmaßnahmen einleiten zu können.

Die Figuren zeigen:
- Fig. 1: ist eine graphische Darstellung, welche das Peptidrückgrat der Raumstrukturen der Superantigene SEB (Fig. 1A), TSST-1 (Fig. 1B) und SEA (Fig. 1C) zeigt. Die Aminosäuresequenzen (SEQ ID NO 1 bis 3) der konservierten β-Faltblatt-Hinge-α-Helix-Domänen und deren Positionen in der Gesamtsequenz sind angegebenen.

## Patentansprüche

1. Verfahren zur Herstellung eines Adsorbens, **gekennzeichnet durch** die Bereitstellung eines Liganden für Bakterientoxine, der ein Oligosaccharid und/oder ein Oligopeptid und/oder ein Oligonukleotid umfaßt und der zur selektiven Wechselwirkung mit der β-Faltblatt-Hinge-α-Helix-Struktur der Superantigene, umfassend die Aminosäuresequenz YNKKKVTAQELD (SEQ lD NO 4), befähigt ist, und kovalente Bindung des Liganden an eine Matrix.

2. Verfahren nach Anspruch 1, wobei das Toxin ein Entero- oder Exotoxin grampositiver Bakterien ist.

3. Verfahren nach Anspruch 2, wobei das Toxin aus der Gruppe, bestehend aus SEA, SEB, SEC, SED, SEE, TSST-1, SPEA und SPEC, ausgewählt ist.

4. Verfahren nach Anspruch 1, wobei die Matrix eine organische Matrix ist.

5. Verfahren nach Anspruch 4, wobei die organische Matrix ein von (Meth)acrylsäureestern und/oder -amiden abgeleitetes Copolymer ist.

6. Verfahren nach Anspruch 5, wobei das von (Meth)acrylsäureestern und/oder - amiden abgeleitete Copolymer Epoxidgruppen aufweist.

7. Verfahren nach Anspruch 5 oder 6, wobei das Copolymer ein durch Polymerisation der monomeren Einheiten
(A) (Meth)acrylamid in einer Menge von 10 bis 30 Gew.-%,
(B) N, N'-Methylen-bis(meth)acrylamid in einer Menge von 30 bis 80 Gew.-% und
(C) Allylglycidylether und/oder Glycidyl(meth)acrylat in einer Menge von 10 bis 20 Gew.-%, jeweils bezogen auf das Gesamtgewicht der monomeren Einheiten,
hergestelltes, statistisches Copolymer ist.

8. Verfahren nach Anspruch 6 oder 7, wobei die Epoxidgruppen vor Einführung der Seitenketten mit Ammoniak oder einem primären Amin aminiert wurden.

9. Verfahren nach einem der Ansprüche 4 bis 8, wobei die organische Matrix aus sphärischen, unaggregierten Partikeln besteht.

10. Verfahren nach Anspruch 9, wobei die sphärischen, unaggregierten Partikel eine Partikelgröße von 50 µm bis 250 µm aufweisen.

11. Verfahren nach einem der Ansprüche 4 bis 10, wobei die organische Matrix eine Ausschlußgrenze von mindestens 10⁷ Dalton aufweist.

12. Verfahren nach einem der Ansprüche 1 bis 11, wobei das Adsorbens biokompatibel ist.

13. Verfahren nach einem der Ansprüche 1 bis 12, wobei das Adsorbens vollblutverträglich ist.

## Claims

1. A method for producing an adsorbent, **characterized by** providing a ligand for bacterial toxins, which comprises an oligosaccharide and/or an oligopeptide and/or an oligonucleotide, and which is capable of selective interaction with the β-sheet-hinge-α-helix structure of the superantigenes, comprising the amino acid sequence YNKKKVTAQELD (SEQ ID NO.: 4), and covalent binding of the ligand to a matrix.

2. The method according to claim 1, wherein the toxin is an entero- or exotoxin of gram-positive bacteria.

3. The method according to claim 2, wherein the toxin is selected from the group consisting of SEA, SEB, SEC, SED, SEE, TSST-1, SPEA and SPEC.

4. The method according to claim 1, wherein the matrix is an organic matrix.

5. The method according to claim 4, wherein the organic matrix is copolymer derived from (meth)acrylic acid esters and/or -amides.

6. The method according to claim 5, wherein the copolymer derived from (meth)acrylic acid esters and/or -amides contains epoxide groups.

7. The method according to claim 5 or 6, wherein the copolymer is a statistical copolymer produced by polymerization of the monomeric units
(A) (meth)acrylamide in a quantity of from 10 to 30% by weight,
(B) N,N'-methylene-bis(meth)acrylamide in a quantity of from 30 to 80% by weight, and
(C) allylglycidyl ether and/or glycidyl (meth)acrylate in a quantity of from 10 to 20% by weight, respectively with regard to the total weight of the monomeric units.

8. The method according to claim 6 or 7, wherein the epoxide groups are aminated with ammonia or a primary amine prior to introduction of the side chains.

9. The method according to anyone of claims 4 to 8, wherein the organic matrix consists of spherical unaggregated particles.

10. The method according to claim 9, wherein the spherical unaggregated particles have a particle size of from 50µm to 250 µm.

11. The method according to anyone of claims 4 to 10, wherein the organic matrix has an exclusion boundary of at least 10⁷ daltons.

12. The method according to anyone of claims 1 to 11, wherein the adsorbent is biologically compatible.

13. The method according to anyone of claims 1 to 12, wherein the adsorbent is compatible with whole blood.

## Revendications

1. Procédé de fabrication d'un adsorbant, **caractérisé par** la disponibilité d'un ligand pour des toxines bactériennes, qui comprend un oligosaccharide et/ou un oligopeptide et/ou un oligonucléotide et qui est capable d'une interaction sélective avec la structure hélice α-charnière-feuillet bêta des superantigènes, comprenant la séquence d'acides aminés YNKKVTAQELD (SEQ ID n°4), et par une liaison covalente du ligand à une matrice.

2. Procédé selon la revendication 1, la toxine étant une entéro- ou exotoxine de bactéries gram-positives.

3. Procédé selon la revendication 2, la toxine étant choisie dans le groupe constitué par SEA, SEB, SEC, SED, SEE, TSST-1n SPEA et SPEC.

4. Procédé selon la revendication 1, la matrice étant une matrice organique.

5. Procédé selon la revendication 4, la matrice organique étant un copolymère dérivé d'esters et/ou d'amides d'acide (méth)acrylique.

6. Procédé selon la revendication 5, le copolymère dérivé d'esters et/ou d'amides d'acide (méth)acrylique présentant des groupes époxyde.

7. Procédé selon la revendication 5 ou 6, le copolymère étant un copolymère statistique fabriqué par polymérisation des unités monomères
(A) du (méth)acrylamide dans une quantité de 10 à 30 %,
(B) du N,N'-méthyles-bis(méth)acrylamide dans une quantité de 30 à 80 % et
(C) de l'éther d'allylglycidyle et/ou du (méth)acrylate de glycidyle dans une quantité de 10 à 20 %, chacun rapporté au poids total des unités monomères.

8. Procédé selon la revendication 6 ou 7, les groupes époxyde ayant été aminés par de l'ammoniac ou un amine primaire avant l'introduction des chaînes latérales.

9. Procédé selon les revendications 4 à 8, la matrice organique étant constituée de particules sphériques non agrégées.

10. Procédé selon la revendication 9, les particules sphériques non agrégées présentant une taille de particule de 50 µm à 250 µm.

11. Procédé selon les revendications 4 à 10, la matrice organique présentant un seuil d'exclusion d'au moins 10⁷ Dalton.

12. Procédé selon l'une quelconque des revendications 1 à 11, l'adsorbant étant biocompatible.

13. Procédé selon l'une quelconque des revendications 1 à 12, l'adsorbant étant toléré dans le sang total.
